# EUROPEAN PATENT APPLICATION

(11) **EP 1 889 547 A2**
(43) Date of publication of application: **20.02.2008**
(21) Application number: 07023043.8
(22) Date of filing: 30.04.1999
(51) Int. Cl.: A23L 1/305, A23C 9/142, A61K 35/20, A61K 38/30, A23C 9/20

(54) **A food composition and method of using same**

(30) Priority: 30.04.1998 AU PP327198
(62) Divisional of application: 99915377.8
(71) Applicant: Numico Research Australia Pty. Ltd., Oakden, S.A. 5086 (AU)
(72) Inventor: Whyte, Peter Bennett Duff, North Haven South Australia 5018 (AU)
(74) Representative: Wright, Simon Mark

(57) **Abstract**

The present invention relates to a food composition preferably for changing body composition and/or physical work capacity. The invention further provides a method of improving body composition and/or physical work capacity. In an aspect of the present invention there is provided a food composition for use in changing the body composition and/or physical work capacity, said food composition including colostrum. In a preferred aspect there is provided a food composition for use in changing the body composition and/or physical work capacity, said food composition including colostrum or a fraction thereof wherein said fraction includes colostrum-derived growth factors maintained therein following fractionation of the colostrum. The food composition may additionally include casein. Preferably the casein is colostrum-derived and is maintained in the colostrum fraction following processing of the colostrum.

## Description

The present invention relates to a food composition preferably for changing body composition and/or physical work capacity. The invention further provides a method of improving body composition and/or physical work capacity.

Colostrum is the first milk secreted by the mammary glands at the end of pregnancy. It differs in composition to the milk produced later. Colostrum is a rich source of growth and anti-microbial factors (Donovan, S. M. and Odle, J (1994)); Foley, J. A. and Otterby, D. E. (1978); Reiter, B. (1978); Shams, D (1994)). Hyperimmune colostrum is commonly used as a source of antibodies against pathogens and has successfully been used to prevent and treat disease caused by pathogens such as rotavirus (Davidson 1989). Colostrum is known to have a role in development of the neonatal gut (Zumkeller, W (1992)) and to stimulate growth of small intestine in newborn piglets (Tungthanathanich, P. R. et al (1992)).

All growth activity cannot be explained by one or other known growth factors alone or in combination. Colostrum is a complex mix of growth factors and other bioactive substances, including (but not limited to) immunoglobulins, lactoferrin, lactoperoxidase, lysozyme, proline-rich polypeptide, and glycoproteins.

One of the many growth factors found in colostrum is IGF-1. In the bovine colostrum, the concentration is more than 20 times greater than in normal milk (Marcotty, C. F. et al (1991); Oda, S. H. et al (1989)). Bovine and human IGF-1 are very similar in composition.

Like many other growth factors, parenterally administered IGF-1 has been shown to have anabolic effects on skeletal muscles (Fryburg, D. A. et al (1995); Tomas, F. M. et al (1991)); and metabolic effects in increasing fat utilisation (Tomas, F.M. et al (1991)). Treatment of idiopathic short stature with human growth hormone relies in part on raising circulating IGF-1 levels. There is no literature linking the use of orally ingested IGF-1 in colostrum and increased height. However, the proteins for which IGF-1 and other growth factors in colostrum increase synthesis is unclear and therefore the anabolic and metabolic effects caused by these factors are not clearly understood. Therefore, all growth activity in colostrum cannot be explained by IGF-1 alone or other known factors.

IGF-1 and other growth factors in their pure forms are expensive. Their use is generally confined to specific medical and pharmaceutical indications including tissue growth and repair. IGF-1 and other growth factors are generally regarded as being rapidly denatured in the gut prior to absorption. Therefore, the effects of the growth factors such as IGF-1, if denatured, would not be expected to be delivered in an active form to the blood or body tissues by an oral route.

Animal studies (Donovan S.M. (1994)) have shown absorption of up to approximately 10% of orally ingested radiolabelled IGF-1 in the first 24 hours after birth. Absorption beyond this period is markedly reduced as the gut "closes" and absorption of macromolecules stops, and as the environment in the gastrointestinal tract changes such that acid and proteolytic breakdown of proteins is more rapid.

Medical benefits of IGF-1 have been obtained via parenteral administration of human IGF. A readily obtainable and useable source of growth factors, high in IGF-1 or other growth factors which has been found suitable to change body composition and/or physical work capacity or to enhance performance in subjects wishing to obtain an improvement of the body such as athletes or for use by patients in a catabolic state/weight loss or for those experiencing fatigue has not been available, nor has there been a convenient or effective means to improve body composition or physical work capacity.

Despite the presence of IGF-1 and other growth factors in colostrum, it has not been proven that IGF-1 and/or other factors from bovine colostrum are available at an effective dose as anabolic agents in humans, nor has colostrum itself been shown or proven to act in an anabolic manner in humans. Therefore, it is generally considered that colostrum would have no benefits on body composition and/or physical work capacity if ingested by humans.

Accordingly it is an object, of the present invention to overcome or at least alleviate some of the problems of the prior art.

In an aspect of the present invention there is provided a food composition for use in changing the body composition and/or physical work capacity, said food composition including colostrum.

In a preferred aspect there is provided a food composition for use in changing the body composition and/or physical work capacity, said food composition including colostrum or a fraction thereof wherein said fraction includes colostrum-derived growth factors maintained therein following fractionation of the colostrum. The food composition may additionally include casein. Preferably the casein is colostrum-derived and is maintained in the colostrum fraction following processing of the colostrum.

The term "physical work capacity" used herein is a measure of the ability to do physical work including any exercise performance, recovery after exercise and reduction of fatigue. The exercise performance may include any one of the exercises selected from the group including (but not limited to) running, walking, jumping, sprinting, knee extensions, knee flexions, squatting, lifting and kicking, resisted and non-resisted exercises and events.

The term "body composition" used herein includes those parameters used to define the make-up of the body including markers of anthropometric or metabolic change (including changes to mood state) selected from the group including (but not limited to) height, percentage body fat, fat mass, fat free mass, dietary intake, oxygen uptake (VO₂max), respiratory exchange ratio (RER), blood, serum creatine kinase (CK), lactulose: rhamnose ratio (L:Rh) intestinal permeability, blood lactate, metabolic and/or respiratory buffer capacity, connective, muscle, nervous and epithelial tissue, and mood state.

The term "changing body composition and/or physical work capacity" as used herein is a change which results in an improvement to the body. An "improvement" may be any change which is favourable for achieving a result, including the perception of an improvement. For instance, an improvement for achieving weight loss or body toning may include a reduction in body fat and an increase in fat-free mass (increased lean tissue and increased loss of fat tissue). Preferably the ratio of fat-free mass to body fat is increased. Preferably the change is an improvement of exercise performance to achieve better athleticism or better endurance in occupational circumstances.

The food composition is preferably for use in changing, in a favourable or improved manner, the body composition and/or physical work capacity preferably in subjects wishing to obtain an improvement to the body as described above, athletes, people with physically demanding occupations or pastimes, or patients in a catabolic state/weight loss situation, or experiencing fatigue. When the food composition is administered to an athlete, it is considered that the food composition including colostrum and a carrier may have a positive effect on the athlete who is subjected to metabolic and physical stresses which normally lead to anthropometric and metabolic adaptations even without supplementation with the food composition.

The food composition may be used by any type of athlete. The athlete may be a power/strength athlete (PSA) or an endurance athlete (EA). The PSA generally undertakes strength and power training as part of their normal training programme. The EA generally undertakes programmes which enhance their ability to undertake prolonged exercise.

The food composition includes colostrum. The source of colostrum may be from any mammal. Preferably the mammal will be selected from the group including (but not limited to) the bovine, ovine, porcine, caprine or equine. More preferably, the colostrum is from the bovine. More preferably the colostrum is collected in the first few days after the end of pregnancy, preferably up to 3 or 4 days after the end of pregnancy.

The colostrum may be treated to reduce contaminants. Preferably the treated colostrum has bioactivity and bioavailability close to that of untreated colostrum.

In a preferred aspect of the present invention there is provided a food composition for use in changing body composition and/or physical work capacity, said food composition including colostrum having a mixture of growth factors and a carrier.

Preferably the growth factors are colostrum-derived and may include IGF-1, preferably derived from colostrum. Pasteurisation, pH changes and some other treatments used to reduce contamination or produce colostrum products can destroy or reduce the levels of growth factors and other bioactive substances found in colostrum, and/or reduce their stability/bioavailability in the gut.

IGF-1 has anabolic effects which may contribute to increased synthesis of contractile or non-contractile proteins which leads to an increase in muscle mass. This may benefit users in terms of increasing muscular power and strength. Preferably, the food composition is for use in changing the exercise performance or physical work capacity in PSA.

However, the only published study to date which has investigated the effect of an orally administered bovine colostrum product is that by Mero, A *et al* (1997). Mero studied the effect of a liquid whey protein extract of colostrum on muscular power as measured by vertical jump performance and failed to find any positive effect on performance. Mero's result supports the commonly held view that orally ingested colostrum containing IGF-1 will not have any effect on performance.

Another commonly held view supported by Mero was that the potential effect of orally administered IGF-1 would be maximised by delivering the whey fraction of colostrum to athletes.

However the results have shown that this did not result in improved performance, showing that the prior art in this field has not yet taught a way to change body composition or work capacity by a means of a food composition.

Contrary to the prior art, the Applicants have postulated that the removal of casein proteins and other manufacturing processes used in producing the whey extract (with high levels of IGF-1) studied by Mero could lead to a loss of intact IGF-1 and other factors in the processed colostrum and less availability of potential active components in the gastrointestinal tract. Casein may contribute to enhancement of bioavailability of IGF-1 and other components of colostrum which lead to changed body composition or work capacity at conditions found in the gut.

Accordingly, the present invention also includes a food composition for use in changing the body composition and/or physical work capacity, said food composition including colostrum or a fraction thereof, wherein said fraction includes growth factors and casein. Preferably the casein and growth factors are colostrum derived and have been retained in the colostrum following processing of the colostrum preferably as described below.

Metabolic effects of parenterally administered IGF-1 show that it increases fat utilisation. It is possible that EA in particular will benefit from this by increasing physical work capacity by having a greater ability to utilise fats for energy during exercise. Increased utilisation of fats may lead to increased glycogen sparing and less lactate accumulation, both of which are associated with a reduction in fatigue and improved performance. Therefore, it is preferable that the food composition is for use in changing the exercise performance or physical work capacity for a EA.

Preferably the concentrations of anabolic growth factors such as IGF-1 are at least the concentrations found in normal untreated colostrum. However, they may be higher. Preferably the growth factors will be in a composition that maximise their availability in the gut, and hence their absorption into and through the gut.

The food composition may also serve as a means of increasing growth factors such as IGF-1 levels in the body. IGF-1 and other factors in the colostrum have been implicated in tissue growth and repair. Their anabolic and metabolic effects may also effect the body composition and physical work capacity. However, if the growth factors are destroyed by digestion in the gut after oral administration, the interplay of factors in colostrum may be unable to contribute to the improved changes in body composition and/or physical work capacity.

The food composition may be supplemented with a carrier. The carrier may support the delivery of the colostrum to the subject as described above. The carrier may be any liquid, solid or semi-solid carrier. It may be a carrier selected from the groups including full cream, skim, modified, flavoured milk, yoghurt including natural, flavoured, frozen or drinking yoghurt, tonics, and sports drinks, other dairy products such as custards, cheese and cottage cheese formulations and ice creams. Semi-solid carriers may be selected from pastes and spreads. Solid carriers may include food bars, biscuits, cereals, food fibres, or any other food.

The food composition may include other supplements beneficial for changing the body composition and/or physical work capacity.

Supplements may be selected from the group including other proteins which may not be found in colostrum, minerals and electrolytes, salts, proteins, amino acids (branched and unbranched), nutrients, lipids, fats, vitamins, carbohydrates (simple and complex), inosine, creatine, HMB, buffering agents and other factors which supplement the diet during training.

Preferably, the minerals may be selected from the group including (but not limited to) calcium, iron, phosphorus, iodine, magnesium, zinc, copper, chromium, molybdenum, and magnesium.

Preferably, the vitamins may be selected from the group including (but not limited to) ascorbic acid (vitamin C), D-Alpha Tocopherol (vitamin E), Niacin (vitamin B3) Riboflavin (vitamin B2), Pantothenic Acid (vitamin B5), Pyridoxine HCl (vitamin B6), Thiamine (vitamin B1), Folic Acid, Cyanocobalamin (vitamin B12), and Cholecalciferol (vitamin D3).

Preferably, the amino acids are selected from the group including (but not limited to) Histidine, Isoleucine, Leucine, Lysine, Methionine, Phenylalanine, Threonine, Tryptophan, Valine, Alanine, Arginine, Aspartic Acid, Cystine, Glutamine, Glutamic Acid, Glycine, Proline, Serine and Tyrosine, and will be chosen to enhance protein synthesis or improve physiological buffering capacity.

Preferably the nutrients are selected from the group including (but not limited to) Biotin, PABA, Choline, Inositol, L-Carnitine, Betaine, and Gamma Oryzanol.

Preferably the salts are selected from the group including (but not limited to) sodium, potassium, and magnesium.

Preferably the carbohydrates are selected from the group including (but not limited to) sucrose, maltodextrose, glucose and fructose.

Preferably the fats are selected from the group including (but not limited to) fat milk solids and vegetable fat. More preferably, fats are retained or reintroduced to the product from the colostrum.

Preferably other factors are selected from the group including emulsifiers such as lecithin, food acids, flavours, preservatives and colourings preferably to improve the delivery and preservation of the food composition.

The food composition may also be provided in any form including a powder, liquid, semi-solid or solid. The food composition may also be delivered as a unit dosage form such as in a tablet, capsule or powder.

In another aspect of the present invention there is provided a method of producing a food composition including colostrum or a fraction thereof including colostrum-derived growth factors maintained therein following fractionation of the colostrum for use in changing body composition and/or physical work capacity, said method including:
providing colostrum prepared by a process including:
   subjecting colostrum to an ultra-filtration process to provide an ultra-filtered colostrum retentate;
   subjecting the ultra-filtered colostrum retentate to a spray drying process; and
   removing the spray-dried colostrum.

The colostrum may be further subjected to a bacterial reduction step preferably utilising centrifugation. The centrifugation may be a flow through centrifugation wherein the centrifugation is performed by controlling throughput and thereby residence time of the colostrum during centrifugation. The centrifugation is preferably performed in conjunction with a low heat treatment (less than 72°C x 15 sec) preferably less than 64°C, thereby retaining the maximum level of bioactivity of active components consistent with food safety requirements.

Standard pasteurisation (72°C x 15 secs) and some other treatments used to reduce contamination or produce colostrum products can destroy or reduce the levels of growth factors and other important bioactive constituents including immunoglobulins and other factors found in colostrum and/or reduce their stability and bioavailability in the gut.

Accordingly, it is preferable that the colostrum is treated by any process to preserve or to enhance the levels of growth factors and other bioactive proteins, lipids or carbohydrates contained therein. Preferably, the colostrum is processed according to the processes outlined in patents AU668033, AU644468, NZ239466, NZ260568 or PCT/AU96/00708, the contents of which are referred to and incorporated herein.

These processes of treating colostrum may further remove salt and contaminants such as bacteria and somatic cells from the colostrum whilst maintaining the bioavailability as close to that of untreated colostrum preferably including casein in the product. Casein has been shown to enhance the bioavailability of IGF-1 at conditions found in the gut lumen. Accordingly, casein may be added or maintained in the colostrum following the processing of the colostrum. The colostrum may be prepared by the following process:
a) the colostrum from up to the first 6 milkings following calving is collected;
b) the colostrum is warmed to 55°C and skimmed in a standard dairy separator to reduce or standardise fat levels; and/or allow the fat portion to be processed separately prior to reintroduction to the product;
c) the skimmed colostrum is then heated up to 63°C for for a period varying from 15 seconds up to 30 minutes and/or centrifuged at 12,000 g in a bactofuge to reduce bioburden and preferably to also improve ability to process downstream;
d) the heat treated skimmed centrifuged colostrum is then either added to fresh dairy or other products ready for consumption with said products acting as a carrier or spray dried ready for consumption or further formulation, or ultra-filtered to reduce water, lactose and electrolyte levels in order to concentrate the protein content or processed in a manner such as ion exhcange chromatography; and
e) the concentrate or eluate is then either added to fresh dairy or other products ready for consumption said products acting as a carrier, or spray dried or processed in a manner such as filtration or UHT, to obtain a long life liquid (LLL) which can also be freeze or spray dried retaining activity of growth factor proteins.

The spray dried powder or LLL can be included in food or pharmaceutical products or taken as is, or reconstituted by the consumer with any suitable carrier. Fat may be reintroduced prior to spray drying.

Preferably the colostrum maintains levels and bioactivity of growth factors similar to that of unprocessed colostrum. The bioactivity can be measured using cell growth assays and making the comparison to untreated colostrum. This may be achieved by utilising the processes outlined in AU668033, AU644468, NZ239466, NZ260568 or PCT/AU96/00708, the contents of which are referred to and incorporated herein. Those processes also maximise the bioavailability of the IGF-1 and other growth factors and immunoglobins by the retention of casein.

In another aspect of the present invention, there is provided a method of changing body composition and/or physical work capacity, said method including administering an effective amount of a food composition including colostrum and a carrier.

Preferably, the food composition is as described above.

The food composition is preferably for use in changing, in a favourable or improved manner, the body composition and/or physical work capacity preferably in subjects wishing to obtain an improvement to the body as described above, athletes, people with physically demanding occupations or pastimes, or patients in a catabolic state/weight loss situation, or experiencing fatigue.

There is also provided a method of increasing tissue mass, said method including administering an effective amount of food composition including colostrum and a carrier.

The tissue may be a tissue selected from the group including connective, epithelial, muscular or nervous tissue. The tissue may be gut tissue or other tissue.

The body composition may change by increasing gut or other tissue mass via an increased synthesis of contractile or non-contractile protein. The anabolic effects of growth factors such as IGF-1 present in colostrum may lead to these increases if provided in a manner which maximises bioavailability of these factors in the gut. A changing of physical work capacity may result from the increased height or muscular power and strength, or increased metabolic or respiratory buffer capacity, or reduced muscle damage.

Also provided is a method of increasing fat utilisation, said method including administering an effective amount of a food composition including colostrum and a carrier.

An increased muscle mass and increased fat utilisation may result in a changing of body composition which includes an increased lean body mass through increased synthesis of proteins due to the anabolic effects of IGF-1 and/or other colostrum factors. Preferably there is reduced fat mass through increased utilisation of fatty acids due to stimulation of fat metabolism by IGF-1 and/or other factors. However, an increased metabolic rate may also result from an increased fat-free mass (FFM). This is particularly helpful for the PSA but also for those wishing to improve their body by weight loss.

Also provided is a method for increasing height, said method including administering an effective amount of a food composition including colostrum and a carrier.

There is also provided a method of reducing physiological fatigue and/or improving the psychological perception of fatigue, said method including administering an effective amount of food composition including colostrum and a carrier.

A reduction in fatigue and/or perception thereof may contribute to a change in physical work capacity experienced through increased muscle mass, or possibly through increased fat metabolism and increased glycogen sparing resulting in less lactate accumulation, or through an increased capacity to buffer the effect of metabolic acids.

Also provided is a method of increasing recovery after exercise said method including administering an effective amount of a food composition including colostrum and a carrier. The increased recovery and/or increased buffer capacity may manifest as an improvement in performance.

There is also provided a method of increasing growth factor and/or other colostrum component levels in the body, said method including administering an effective amount of food composition including colostrum and a carrier.

The increased growth factor or other colostrum component levels may result in a change to the body composition and/or work capacity.

Preferably, the increased growth factor component levels are provided by the colostrum (colostrum-derived).- Increases in growth factors in particular may contribute to any of the changes in body composition and/or physical work capacity mentioned herein. Preferably, the growth factors include IGF-1.

The levels of IGF-1 may increase in the body, preferably in the gut. Levels of IGF-1 in the blood have generally been shown not to increase significantly after oral administration. However, maximum bioavailability is achieved by the composition herein described, which has overcome some of the problems of obtaining improved work capacity/body composition.

Despite the commonly held belief that IGF-1 and other macromolecules are quickly digested in the gastrointestinal tract, and that orally ingested growth factors such as IGF-1 are not available systemically, oral administration of colostrum as described above has been demonstrated by the Applicants to result in improvements in body composition and work capacity.

There is also provided a method of treating or preventing a disorder of the gut, said method including administering an effective amount of a food composition including colostrum and a carrier.

A disorder of the gut may be selected from the group including mucositis, gastrointestinal damage from administration of non-steroidal anti-inflammatory or other drugs, gastrointestinal damage from irradiation therapy, gastrointestinal damage from chemotherapy, damage from infection caused by pathogens such as rotavirus, *E. Coli spp, Salmonella spp, Cryptosporidium spp, H. pylori* etc, (eg. in, but not limited, to HIV/AIDS patients), damage from gut surgery, and damage due to disease such as crohn's disease, inflammatory bowel syndrome, coeliac disease or cystic fibrosis.

There is also provided a method of facilitating gut growth and development said method including administering an effective amount of a food composition including colostrum and a carrier.

The use of the food composition may be particularly for use in treatment of short bowel syndrome, massive small bowel resection (MSBR) and/or intestinal adaptation resulting from the residual bowel compensating for a loss in absorptive surface. The treatment may further include the prevention of side effects such as diarrhoea caused by short bowel syndrome, MSBR or intestinal adaptation.

Preferably the food composition for gut growth and development includes colostrum prepared by the processes described above. A preferred dose may be in the order of 1 to 10 g/kg/day.

The treatment may be conducted by using the food composition alone or in combination with other additional growth factors such as Glucagen-like peptide (GLP-2) analogue, and/or Insulin-like growth factor (IGF-1).

In another aspect there is provided a method of increasing buffering capacity, said method including administering an effective amount of food composition including colostrum and a carrier.

The addition of colostrum may buffer against effects of lactic acid in and during exercise. When lactic acid builds up, the food composition may be administered before, during or after exercise as a preventative measure.

In yet another aspect, there is provided a method of reducing effects of metabolic acidosis, said method including administering an effective amount of a food composition including colostrum and a carrier

Again, the presence of a colostrum based food composition may be administered before, during or after exercise or training.

The colostrum may be hyperimmune colostrum from animals vaccinated in order to produce antibodies effective in preventing or treating infectious diseases. Athletes undergoing heavy training programmes are at risk of infections due to lowered immune status and by often travelling.

Endurance athletes sometimes suffer bouts of diarrhoea which are induced by their training. Damage to the intestinal mucosa is known to increase intestinal permeability and cause diarrhoea [Ford, R.P.K. et al (1986)] and although the precise mechanism of the diarrhoea experienced by endurance athletes is unknown, it is generally accepted that mechanically induced damage to the intestinal mucosa resulting from repetitive compression of the gut during long training sessions increases intestinal permeability and leads to episodes of diarrhoea.

There is also provided a method of reducing muscle damage during exercise, said method including administering an effective amount of a food composition including colostrum and a carrier.

Creatine kinase is present in skeletal muscle, brain tissue and heart tissue and damage to these tissues results in the release of increased levels of creatine kinase into the blood. Plasma creatine kinase concentrations are sometimes elevated during exercise, indicating that the exercise has induced muscle damage. The increased IGF-1 and/or other factors provided in colostrum may contribute to an increase in protein structure of muscle and thereby preventing against injury, or may improve wound repair, thereby contributing to the reduced muscle damage during exercise.

In a preferred aspect there is provided a method of changing body composition and/or physical work capacity said method including administering over an effective period, an effective amount of a food composition including colostrum and a carrier.

Preferably, the food composition is as described above and prepared by the methods outlined above.

The term "effective period" is a period wherein a significant improvement in body composition and/or physical work capacity is noticed. The period may be up to 4 weeks or up to 8 weeks. However, the period may be longer depending on the condition to be improved.

The term "effective amount" will depend on the subject undergoing the improvement and on the bioactivity of the factors in colostrum, and on the bioavailability of those factors in the gut. Preferably, the subject is a PSA or a EA or someone who might benefit from reduced muscle damage, increased physiological buffering capacity, increased height, perception of reduced fatigue during training, improved recovery and/or performance, improved body composition. For colostrum preferably prepared as described herein, the daily dosage may equate to approximately 0.2-2 g/kg per day. Casein has been shown to be very effective for preserving IGF-1 at conditions found in the gut. Where casein is absent, the equivalent amount of IGF-1 or other factors in colostrum required for an effective amount may be approximately 4 to 5 times the dose.

In determining the dosage of colostrum, consideration should also be given to the quantity of colostrum that could reasonably be consumed for a day. Colostrum powder preferably prepared as described herein may be dissolved in any carrier or liquid, semi-solid or powder form as described above. Skimmed milk or a skimmed milk/water mix may be used so that it can be taken orally as a food drink. 40 g of colostrum powder can be adequately dissolved in 250 mL of skimmed milk/water. It is preferable that the subjects (patients or athletes) be given a dosage of approximately 0.5-1 g/kg per day. Preferably the colostrum has been prepared by the above mentioned processes, preferably for retaining casein in the colostrum.

The colostrum may be delivered to the subject in a single dose or in multiple doses over each period of 24 hours. For example, for a 60 g dosage of colostrum, 20 g of bovine colostrum powder may be provided to the subject in morning and 40 g of bovine colostrum powder may be provided in the evening.

In a further preferred aspect there is provided a method of changing body composition and/or physical work capacity in an athlete, said method including administering to an athlete an effective amount of a food composition including colostrum and a carrier.

Preferably the colostrum is prepared as described herein.

Preferably, the athlete is a PSA or an EA. The two types of athletes have different demands on their training which leads to differences in the way their bodies adapt.

The changed body composition may be a change measurable by comparing anthropometric measurements over a period of time, preferably over a period of at least 4 weeks, more preferably over 8 weeks. Preferably, the anthropometric measurements are selected from the group including height, mass, skinfolds, thigh girths, calf girths, leg volumes, and body composition by hydrodensitometry, or plethysmography. Therefore it is preferred that the method is applicable to changing any one of these anthropometric measurements, or a metabolic change such as physiological buffering capacity, or mood state.

The changed physical work capacity may be measured by comparing an athletes performance of a battery of exercises over a period of time, preferably over a period of at least 4 weeks, more preferably, over 8 weeks. Preferably, for a PSA, the battery of exercises may be selected from the group including measuring the effect on a cycle, knee extensions, and/or knee flexions, vertical jump heights and sprint accelerations. Preferably, for an EA, the test battery may be selected from the group including but not limited to measurement of an exercise test more preferably an incremental exercise test to determine total times and/or distances covered to exhaustion, total work done, peak oxygen uptakes, oxygen uptake and heart rates at lactate threshold and respiratory exchange ratios (RER), buffer capacity, and blood lactate concentrations during submaximal exercise.

Therefore, it is preferred that the method is applicable to changing any one of these measurements. Most preferably there is provided a method of improving performance in an EA, said method including administering over an effective period, an effective amount of a food composition including colostrum and a carrier.

Preferably, the colostrum is prepared as described herein.

Also it is preferable to provide a method of reducing serum creatine kinase (CK) levels in PSA or an EA or other person likely to incur muscle damage as a result of exertion or exercise, said method including administering over an effective period, an effective amount of a food composition including colostrum and a carrier.

Preferably the colostrum is prepared as described herein.

The reduced levels of serum CK may be a result of reduced muscle damage. Accordingly, there is also provided a method of reducing muscle damage by administering to an athlete an effective amount of a food composition including colostrum and a carrier.

Administration may be via any route which does not cause side effects to the body. For instance, colostrum contains high levels of proteins which may cause immune responses. Therefore, injection is not a suitable means of administration. However, other means including oral, rectal, or dermal administration is suitable providing sufficient colostrum can be delivered to the body.

Administration may be staggered or continuous. By "staggered", it is meant that a dose could be delivered at intervals during the day. By "continuous" the colostrum may be delivered to the body via a pump or other continuous delivery means either to the mouth or directly to the gastrointestinal tract.

During a period of training the food composition including colostrum and a carrier may be administered before and/or during exercise.

Colostrum may be administered after exercise. If hyperimmune colostrum is used, administration before and during exercise could help prevent infection. If administered after, the colostrum could help treat infection and gut damage by combined activity of antibody and growth factors.

The present invention will now be more fully described with reference to the following examples. It should be understood, however, that the description following is illustrative only and should not be taken in any way as a restriction on the generality of the invention described above.

### FIGURES

Figures 1A and 1B show that physiological buffer capacity was lower in the Placebo group compared with the Active group during both the first and second rows at week 9 (P=0.02).

Figure 2 shows the difference between the distance covered between an Active and a Placebo group in a maximal rowing test.

Figure 3 shows the difference between the work done between an Active and a Placebo group in a maximal rowing test.

Figure 4 shows a difference in the change in stature between the Active and Placebo group.

Figure 5 shows a difference in fatigue perception between an Active and Placebo group.

Figure 6 shows a difference in fat mass between an Active and Placebo group.

### EXAMPLE 1

### Testing Procedures to monitor changes in body composition and physical work capacity

### A. Growth

### 1. Body Mass and Stature (height)

Body mass was measured using a set of electronic digital scales (AND Mercury, FV-150). Stature was assessed using a stadiometer (SECA).

### 2. Resting Blood Pressure (RBP)

RBP was monitored with subjects in a seated position using a manual sphygmomanometer (Accoson MK 2).

### 3. Thigh and Calf Girths

Thigh and calf girths were measured using a metal tape (Lufkin, W606PM) with subjects standing in the anatomical position. Thigh girths were measured at the midpoint between the greater trochanter and the tibial plateau. Calf girths were measured at the point of greatest circumference.

### 4. Fat Mass (FM) and Fat-Free Mass (FFM)

FM and FFM were determined by calculating body density (BD) using hydrodensitometry (underwater weighing) according to the following method:
A chair was suspended by a pulley system from a Western Load Cell connected to a linear chart recorder (Rikadenki). The load cell was calibrated using a 5 kg calibration mass. A minimum of 5 underwater trials were conducted at residual volume and the largest value recorded was taken to be the underwater mass. Water temperature was maintained in the range 30.2° - 36.1° C.

The residual volume was measured by helium dilution using a 10 litre Stead-Wells modular spirometer and helium analyser; 125 mL was subtracted from the functional residual capacity in order to correct for the deadspace of the mouthpiece and the volume of helium absorbed by the blood. The residual volume was measured after the underwater trials. Measurements were recorded whilst the subjects was immersed to neck level and in the same posture as during the underwater trials. BD was then determined from the formula of Goldman and Buskirk , except that no correction was applied for gas in the gastrointestinal tract; percentage body fat (%BF) was then calculated according to Siri.

There was no significant change in residual volume in either group of subjects throughout the duration of the study (P>0.05) so the lowest residual volume recorded for each subject was used for the calculation of BD, %BF, FM and FFM.

The coefficient of variation for the determination of BD using the above method was 0.02%.

### 5. Blood Sampling Techniques

After being underwater weighed the subjects dried themselves and changed into their exercise clothing. A venous blood sample (10 mL) was then taken from the median cubital vein at the elbow of one arm. 6 mL of blood was placed into a plastic tube and allowed to clot in order to obtain serum. The remaining 4 mL was placed into a tube containing dipotassium EDTA as an anticoagulant to obtain plasma. Both tubes were then centrifuged for 10 min at a relative centrifugal force of 2000 g at 4°C in a refrigerated centrifuge (Beckman, GS-6R). The serum was then drawn off and divided evenly amongst 3 plastic test tubes and frozen at -20°C for subsequent analysis of serum creatine kinase (CK) concentrations in triplicate (see below). The plasma was drawn off and 1 ml was frozen at -20°C for subsequent analysis of plasma IGF-1 concentrations (see below), whilst the remainder was frozen at-20° C in a plastic test tube and retained in case subsequent analysis proved necessary.

### 6. Determination of Plasma IGF-1 Concentrations

The plasma concentrations of IGF-1 in each sample were measured three times by radioimmunoassay after separation of their binding proteins by high performance size exclusion liquid chromatography at pH 2.5 according to the method as modified by Owens et. al. 1990; 1994. The mean of the three assays of each sample was taken as the plasma concentration.

This procedure provided an average within-assay coefficient of variation of 10% and a between assay coefficient of variation of 16%.

### 7. Determination of Serum Creatine Kinase Concentrations

The serum concentrations were measured in triplicate with an Hitachi 917 automatic analyser using a zero order kinetic assay. The mean of the triplicate assays was taken as the serum concentration.

The coefficient of variation for this assay was 2.4% at 172 U/L and 1.2% at 485 U/L.

### B. Supplement Preparation

The bovine colostrum used as the supplement in the present study was prepared according to the patented processes as outlined in Australian Patents 644468, 668033 and New Zealand Patents 239466, 260568 the contents of which are incorporated herein by reference.

The colostrum from up to the first six milkings following calving was collected.

The colostrum was warmed to 55°C and skimmed in a standard dairy separator to reduce fat.

The skimmed colostrum was then heat treated at no more than 63°C for no more than 30 min and/or centrifuged at 12, 000 g in a Bactofuge to reduce bioburden.

The pasteurised colostrum was then ultrafiltered to reduce water, lactose and electrolyte levels in order to concentrate the protein content.

The concentrate was then spray dried in an aseptic, pharmaceutical model 2 stage spray drier with a filtered air supply to produce colostrum powder (powder production accords with the Code of Good Manufacturing Practice for Therapeutic Goods in Australia).

The supplements were mixed with skimmed milk/water and taken orally.

The placebo used in the study was whey protein concentrate (Alacen 372, New Zealand Dairy Corporation).

The supplements were provided in 20 g sachets and the subjects consumed the contents of one sachet (20 g) each morning with their morning meal, and two sachets (40 g) with their evening meal. Each subject prepared the powder for consumption in the following manner:
Each 20 g sachet of powder was added to 85 mL of warm water and mixed thoroughly;
40 mL of skim milk was then added for each 20 g of powder and the mixture was shaken until well mixed.
If desired the mixture was refrigerated until chilled before drinking, or it could be drunk warm.

### EXAMPLE 2

### Effects of oral colostrum supplementation on Endurance Athletes (EA) - Running Performance

The following study was conducted to determine whether bovine colostrum has favourable effects on running performance in Endurance Athletes (EA). For the present study, bovine colostrum powder as prepared above was used as a supplement in the food composition ingested by the athletes.

The purpose of this study was to determine whether the oral administration of 60 g per day of bovine colostrum powder (intact^{™}) was more effective than the administration of 60 g per day of whey protein powder in:
1) improving endurance running performance
2) reducing body fat
3) reducing exercise induced muscle damage

The present study employed a double-blind, placebo controlled, parallel, randomised design to determine the effect of supplementation with a low fat, low lactose, concentrated bovine colostrum protein powder (intact^{™}, NorthField Laboratories Pty Ltd) on plasma IGF-1 concentrations and endurance running performance. After an initial familiarisation period in the two weeks prior to commencement, 39 males, aged 18-35 years, completed an 8 week running program (3 x 45 minutes/week at lactate threshold) whilst consuming 60 g/day on intact^{™} bovine colostrum (n=23, peak VO₂ 53.5 ± 1.1 ml.kg¹ min¹) or whey protein (n=16, peak VO₂ 54.2 ± 1.7 ml.kg¹ min¹). All subjects followed dietary guidelines provided by the researchers and kept food diaries throughout the study period for subsequent dietary analysis. Subjects completed 2 incremental treadmill running tests to exhaustion (10 km/hr, incremented 1% grade every 3 min) separated by 20 minutes of recovery at weeks 0, 4 and 8. There were no differences in plasma IGF-1 concentrations between the groups at week 0 (colostrum 231.1 ± 10.7 ng/ml, placebo 221.0 ± 13.3 ng/ml; P=0.37). Plasma IGF-1 concentrations did not change in either group during the study period (P=0.90). There were no differences in the distance covered (m) or work done (kJ; vertical distance covered x body mass x 9.81 m/s²) during the first (colostrum 4649 ± 238 m, 155.8 ± 15.7 kJ; placebo 4464 ± 320 m, 140.2 ± 19.6 kJ; P>0.46) or the second (colostrum 4044 ± 357 m, 120.6 ± 21.3 kJ; placebo 3942 ± 388 m, 110.7 ± 21.1 kJ; P>0.91) treadmill runs at week 0. Distance covered and work done during the first treadmill run increased in both groups during the study period (P<0.01), but at similar rates (P>0.69). During the second treadmill run both groups exhibited similar increases in the distance covered and work done from weeks 0-4 (P>0.20) but, from weeks 4-8 the intact^{™} colostrum group continued to improve whilst the performance of the placebo group plateaued, such that by week 8 the colostrum group ran further (colostrum 4662 ± 251 m, placebo 4237 ± 323 m; P=0.04) and did more work than the placebo group (colostrum 150.7 ± 17.1 kJ, placebo 124.2 ± 18.9 kJ; P=0.03) The TEM for running time (which equates to distance covered and work done) was 2%. There were no differences in dietary intakes between two groups. These results indicate that oral supplementation with intact^{™} bovine colostrum improves the ability to perform a second bout of maximal exercise following a relatively short period of recovery from a prior bout of maximal exercise.

This study confirmed significantly enhanced ability to run and work *after* the rest period. Strong trends are shown towards less muscle damage as measured by creatine kinase.

In a study that measured fat mass and fat free mass, post-hoc analysis showed that fat mass had decreased significantly in the active group (P=0.005) but not in the placebo group (P=0.08) by Week 8.

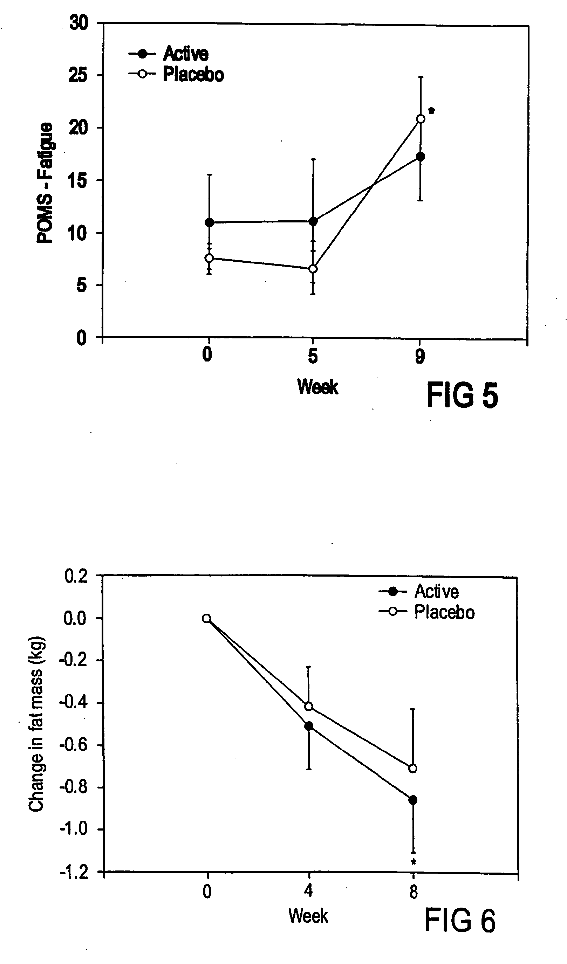

Overall there was also a trend to reduced fat mass despite the intact^{™} colostrum group having started the test period with lower fat mass % than the placebo group (see Figure 6).

Throughout the period of the study there was a trend for the athletes taking the colostrum supplements to increase the distance covered during the first of the two treadmill runs at each trial. However, the athletes taking the colostrum supplement did demonstrate significantly greater increases in the distance covered and the amount of work done during the second treadmill runs at each trial. As a result of the greater increase in the work done during the second treadmill runs at each trial there was also a significantly greater increase in the total work done during both runs at each trial in the colostrum group.

In some cases, the finding of no significant difference in performance between the two groups during the first treadmill run at each trial (although there was a trend suggesting that performances were improved) but a significantly greater improvement in the colostrum group during the second run suggests that although there may be some performance enhancing effect of colostrum supplementation, there is apparently a greater effect in terms of improving recovery from previous exercise. The mechanism for this enhanced recovery is not immediately apparent from the data collected in the present study since there were no differences in blood lactate or RER responses during the second treadmill runs between the two groups at any of the trials. There was however, a trend for the CK values to be less elevated at the end of each trial in the colostrum group and it is possible that a reduced level of muscle damage caused in the first treadmill run could have assisted the subjects on the colostrum supplement in sustaining performing better in the second treadmill run. However, it cannot be ruled out that some other parameter which was not measured could have led to the greater improvement in these subjects.

There was no change in plasma IGF-1 concentrations in response to taking bovine colostrum supplements, despite the fact that it has been shown that orally administered ¹²⁵I-IGF is transported into the circulation in calves

In summary, this study provides evidence that oral supplementation with bovine colostrum can enhance increases in endurance capacity and body composition beyond those which will ordinarily be achieved through training alone.

### EXAMPLE 3

### Effects of oral colostrum supplementation on Endurance Athletes - Elite Rowing Performance (female)

The following study was conducted to determine whether bovine colostrum has favourable effects on rowing performance in Endurance Athletes. Bovine colostrum powder as prepared above was used as a supplement for the food composition ingested by the athletes.

### (a) Physiological Buffer Capacity

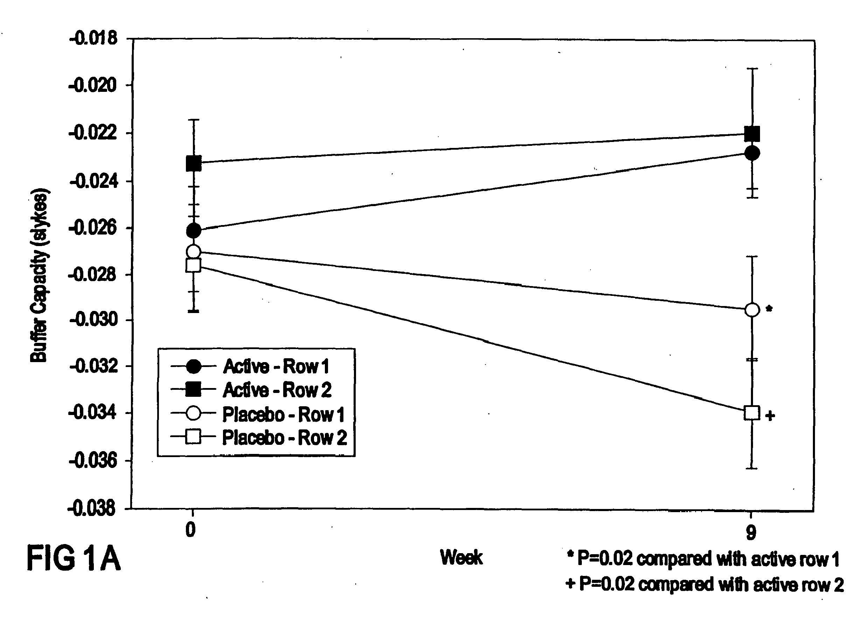

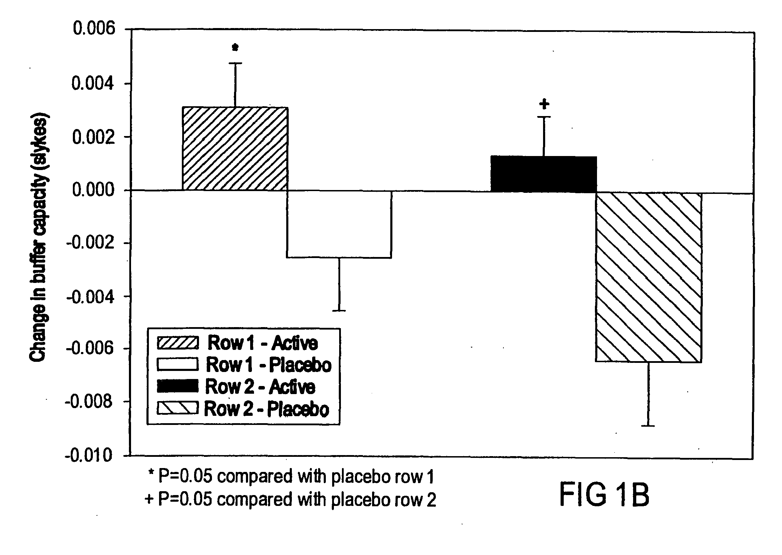

Results in Figures 1A and 1 B show that colostrum had a protective effect on buffer capacity during a second row. Physiological buffer capacity was lower in the placebo group during the first and second rows at week 9 (P=0.02). This was due to a tendency for an increase in buffer capacity in the Active group and a decrease in the Placebo group such that there was a significant difference in the change in buffer capacity between the two groups (P=0.05).

The Active group also demonstrated an ability to maintain or enhance their capacity to buffer against the effects of lactic acid in blood pH by week 9 of the study during rowing exercise (P=0.05) such that their blood pH dropped less for a given increase in lactic acid concentrations.

A lesser ability to buffer effects of lactic acid may result in an increased susceptibility to fatigue. Hence the placebo group did not perform as well as the active group.

Buffer capacity is calculated from the relationship between blood lactate and pH and represents the drop in pH per unit increase in lactate. Buffer capacity reflects the ability of the body's buffer systems to counteract the pH decreasing effects of lactic acid.

### (b) Exercise Performance Improvement

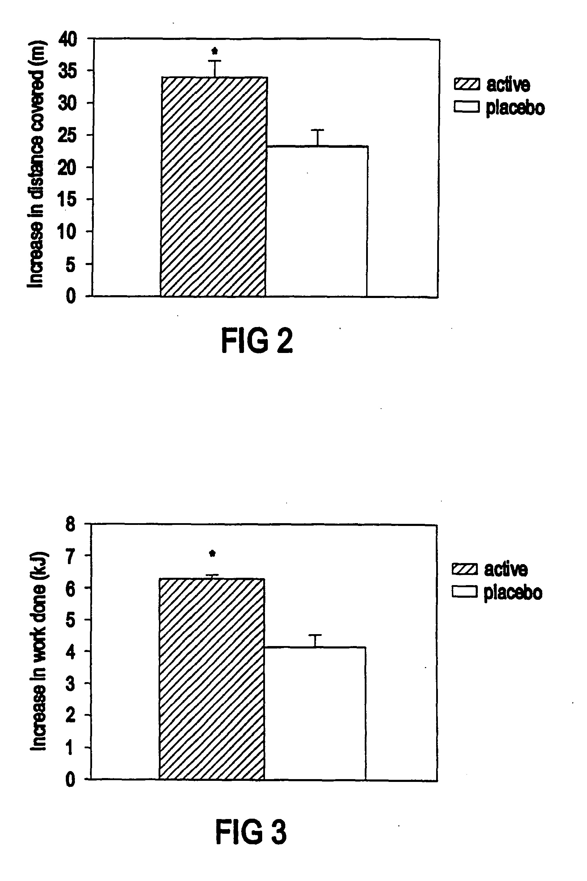

Results in Figures 2 and 3 demonstrate that the active group had a significantly greater improvement in distance covered (P=0.05) and work done during a maximal rowing test.

### (c) Growth

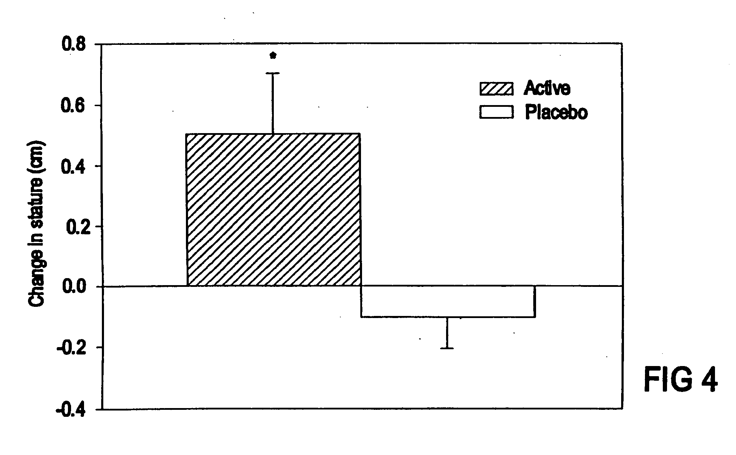

The subjects on the colostrum grew more than those on the placebo. The Active group were taller than the Placebo group at week 0 (Active 176.5 ± 1.1.cm, Placebo 172.5 ± 1.65 cm; P=0.0002) but grew more than the Placebo group during the study period (P=0.02). The greater increase in height could not be accounted for by either the initial height (r²=0.06, P=0.53), nor the age (r²=0.06, P=0.54) of the subjects at week 0. Results are shown in Figure 4.

The results point to growth factors being absorbed from the colostrum and opens up possibilities for further studies into growth promotion in children.

### (d) Fatigue Perception

Psychological perception of fatigue as determined using a Profile of Mood States (POMS) questionnaire remained relatively stable during the first 5 weeks of study, but during the final 4 weeks the observed increase in fatigue perception was attenuated in the Active group (P=0.05). Results are shown in Figure 5.

In summary, rowers ingesting intact^{™} colostrum demonstrated:
- improved ability to perform a maximal workload;
- a significant improvement in physiological buffering capacity (measured in blood samples) during the second rowing exercise after the rest period, which is not explained by bicarbonate levels. The difference in buffering capacity is a new finding that help to explain the performance improvements being seen, and has potential to benefit anyone with a need to cope with metabolic acidosis (sports due to lactic acid, other causes of acidosis);
- a significantly greater increase in height during the study period. This is a new finding which is unexpected, as it against suggests that there is a systemic effect of taking intact^{™} over and above the nutritional benefit of the placebo
- a perception of less fatigue during training as measured by a Profile of Mood States (POMS) questionnaire.

### EXAMPLE 4

### Effects of oral colostrum supplementation on Power/Strength Athletics - Vertical Jump, Peak power and force

The following study was conducted to determine whether bovine colostrum has favourable effects on vertical jump, peak power and peak force performance in Power Strength Athletes (PSA). For the present study, bovine colostrum powder as prepared above was used as a supplement in the food composition ingested by the athletes.

The present study employed a double-blind, placebo controlled, parallel, randomised design to determine the effect of 8 weeks of supplementation with bovine colostrum powder (intact^{™}, NorthField Laboratories Pty Ltd) on plasma IGF-1 concentrations and a number of functional measures of muscle power output.

After an initial familiarisation period in the two weeks prior to commencement, 51 males, aged 18-35 years, completed an 8 week resistance and plyometric training program (6 days per week) whilst consuming 60 g/day of intact^{™} bovine colostrum (n=26) or whey protein (n=25). All subjects followed dietary guidelines provided by the researchers and kept food diaries throughout the study period for subsequent dietary analysis. Subjects performed a battery of four exercise tests twice at weeks 0, 4 and 8 of the study, with each performance of the test battery separated by 20 min of recovery. The test battery consisted of 3 x 20 m sprints, 3 x 10 second bouts of maximal cycle exercise (MCE), 3 x maximal vertical jumps (VJ) and 3 x maximal knee extensions (KE) and knee flexions (KF) on an isokinetic dynamometer. The best of the 3 attempts at each exercise was recorded for each performance of the test battery.

There was no difference in plasma IGF-1 concentration between the groups at week 0 (colostrum 255.8 ± 15.5 ng/ml, placebo 259.7 ± 23.0 ng/ml; P=0.87) and plasma IGF-1 did not change in either group during the study period (P=0.58). The training volumes completed by each of the two groups were the same for both the resistance (P=0.37) and plyometric programs (P=0.57). By week 8 the colostrum group had improved their VJ significantly more than the placebo group during both the first (colostrum 3.0 ± 0.6 cm, placebo 1.3 ± 0.7 cm; P=0.004) and second (colostrum 2.5 ± 0.6 cm, placebo 0.8 ± 0.7 cm; P= 0.002) performances of the test battery. There were also trends for the colostrum group to exhibit greater improvements in absolute and relative peak power outputs during MCE (P=0.09) and peak force generated for KF (P=0.07). Creatine kinase (CK) activity tended to increase less in the colostrum group (P=0.14), particularly during the first 4 weeks of the study (P=0.06).

These results indicate that oral supplementation with intact^{™} bovine colostrum facilitates a greater improvement in maximal power output in response to the same training program. Sortie of the greater performance improvement may be attributable to a reduction in muscle damage.

This study confirmed significantly enhanced ability to perform vertical jump and trends to improve peak power and peak force. There was a strong trend towards reduced muscle damage as measured by creatine kinase.

The principal finding of the present study was that in athletes undertaking a power training program oral supplementation with bovine colostrum prepared as above resulted in a significantly greater increase in vertical jumping performance than was achieved using an oral protein supplement.

The only other study which has investigated the effect of bovine colostrum supplementation on the expression of muscle power [Mero, A. et al (1997)] found that colostrum supplementation resulted in a significant difference in serum IGF-1 concentrations, but had no effect on muscle power, as measured using vertical jump displacement. The active component in bovine colostrum which is most likely to increase muscle power is IGF-1, since this hormone is known to have anabolic effects on skeletal muscle [Fryburg D.A. et al (1995); Tomas, F.M. et al (1991) (a) and Tomas, F.M. et al (1991)] However, despite reporting a significant difference in serum IGF-1 concentrations, Mero et. al. found no improvement in vertical jumping performance. In the present study the reverse was true, no detectable increase in plasma IGF-1 concentrations were observed, but there was a significant improvement in vertical jumping performance.

During the study there was no significant increase in pre-exercise serum CK concentrations indicating that the training did not induce significant amounts of muscle damage in either group. However, despite the lack of statistical significance there was a trend for serum CK values to increase in the placebo group, but not in the colostrum group

In summary, colostrum prepared as above significantly improved vertical jumping performance in PSA.

### EXAMPLE 5

### Effects of oral colostrum supplementation on Pigsgrowth of gut and body

Growth factors are present in relatively high quantities in colostrum and play an important part in gut development. Gut development and growth in the piglets is integral to efficient nutrient absorption and protection against bacterial invasion. The growth factor content of milk decreases with advancing lactation and so the opportunity exists to enhance gut growth and development through supplementation with exogenous growth factors. This is particularly pertinent given that the pig gut is relatively immature at weaning and the pig suffers a growth check at or around this time. Recent data has shown that systemic treatment of both by artificially rearing pigs with growth factor analogues increases liver weight and gut growth and development. However, if orally consumed, growth factors may be destroyed by digestion.

Piglet growth can also be limited by the amount of milk they obtain from the sow. Recent research has shown that piglets allowed to consume cows milk *ad libitum* grow approximately twice that of suckled piglets (230 vs 450 g/d). Therefore, early weaning combined with artificial rearing or creep feeding may be a means of increasing piglets growth.

### Diets

Two diets were used in this experiment - one containing intact^{™} colostrum protein concentrate (NorthField Laboratories) prepared by the process of Example 1 and the other, a whey protein concentrate. The diets were formulated to contain equal levels of crude protein and amino acids: It was assumed that the availability of amino acids in the whey protein concentrate and the colostrum protein were equal. The diets were protein deficient so that the efficacy of any growth factors would be clearly demonstrated. Lactose and oil was being kept relatively constant in the diets.

To prepare the diets, the butter, oil and water were heated to 70°C. All dry ingredients were then added to the water and mixed together with 1 ml of anti-foaming agent. The melted butter and oil was then mixed with the water. To ensure even distribution of the oil throughout the mix, the milk supplement was homogenised.

Following homogenisation, the vitamins and minerals were mixed into the supplement and it was frozen until required.

### Pigs

Sixteen large white male and female piglets were weaned from sows at one day of age. Piglets were allocated to one of two diets based on eight pigs per diet (4 male, 4 female). Piglets were identified with coloured ear tags.

Piglets were trained over two days to suck the liquid diets from a lambar teat. The diets were offered *ad libitum* and represented the only source of nutrients and fluids (i.e. no additional water was supplied). The liquid diets were always fresh and were replaced twice per day. Milk intake and rejects was recorded twice daily. Piglet weights were recorded weekly.

The pigs were offered their diets until they were four weeks of age. At this time 6 males and 6 females from each treatment were euthanased and the gut contents emptied. The weight of the full gut, empty stomach, empty small intestine, empty caecum and empty large intestine were recorded. Small intestine and large intestine lengths were measured. Heart and lung, liver and spleen weights were also recorded.

### Results

### Growth rates and feed conversion ratio

Addition of the intact^{™} colostrum to a sows milk supplement significantly improved (P<0.01) the four week weight and overall growth rate of piglets from birth to four weeks of age compared to those fed a sows milk supplement containing whey protein.

Significant differences in overall growth rate were not evident until week three of the experiment although the piglets fed sows milk supplement containing intact^{™} colostrum had a significantly higher growth rate for weeks two and three respectively.

Addition of the intact^{™} colostrum to a sows milk supplement significantly improved (P<0.05) the overall feed conversion ratio of piglets from birth to four weeks of age compared to those fed a sows milk supplement containing whey protein.

### Intestine length

Addition of the intact^{™} colostrum resulted in a significant increase in the total intestine length (P<0.05), small intestine length (P<0.05) and large intestine length (P<0.0.1). Piglets fed the sows milk supplement containing the intact^{™} colostrum had a significantly (P<0.05) lower small intestine length relative to the whole intestine length and a significantly higher (P<0.05) large intestine length relative to the whole intestine length.

### Digestive tract weights

Piglets fed the sows milk supplement containing the intact^{™} colostrum had a significantly higher (P<0.05) full gut weight, empty gut weight, stomach weight, small intestine weight and large intestine weight compared to piglets fed a sows milk supplement containing whey protein.

The ratio of large intestine weight to empty gut weight was significantly increased (P<0.01) in piglets fed the sows milk supplement containing the intact^{™} colostrum.

The ratio of empty gut, stomach, small intestine, caecum and large intestine to empty-body-weight respectively was not significantly different (P>0.05) between piglets fed sows milk supplements containing either the intact^{™} colostrum or the whey protein concentrate.

### Organ weights

Liver weights were significantly higher (P<0.05) in piglets fed the sows milk supplement containing the intact^{™} colostrum. No significant differences were detected (P>0.05) between the weight of the heart and lungs and the spleen and the relative proportions of all organs to the empty body weight.

### Discussion

Growth rates and gut development in piglets were promoted from birth to four weeks of age by the addition of the intact^{™} colostrum to a sows milk supplement. As the intact^{™} colostrum was compared with a whey protein concentrate in diets that were formulated to contain equal levels of crude protein and amino acids, it is likely that the improvement growth was due to the activity of growth factors present in the colostrum prepared as in Example 1. The activity of these growth factors may be confirmed by analysis of blood samples taken during the course of the experiment.

The growth rates observed were similar to those reported previously for piglets suckling the sow and significantly less than those observed for piglets offered cows milk *ad libitum* (450 g/day). As the sows milk supplements used in this experiment were offered *ad libitum,* it can be concluded that the diets were highly protein deficient. This is likely to have contributed to the expression of the growth factors present in the intact^{™} colostrum. Despite the low protein content of the supplements, the ability to achieve a four week weight of 8.1 kg or greater would be highly valued by the commercial pig industry.

The increase in the empty gut, stomach, small intestine and large intestine weight can be largely attributed to the greater body weight of these piglets. The enhanced development of the large intestine in piglets fed the intact^{™} colostrum, however, would give these piglets a greater ability to adapt to solid diets with a significantly higher fibre content. This in turn would reduce the post-weaning check observed when piglets are weaned from liquid to solid feed.

Improvements in FCR in piglets fed the intact^{™} colostrum are likely to be due to the increased size of these piglets and a greater gut capacity. The absolute increase in liver weight is likely to be due to a greater body weight in pigs fed the intact^{™} colostrum rather than due to dietary influences. Had the growth factors in the isolate had any effect on liver size and function, it is likely that we would have observed an increase in the proportion of liver weight to empty body weight.

Finally, it is to be understood that various other modifications and/or alterations may be made without departing from the spirit of the present invention as outlined herein, and that these results will have application in patient groups outside athletes such as, but not restricted to, infant nutrition, catabolic states, gut growth and development diseases of the gut, short stature, metabolic acidosis, gut health repair and fatigue, including chronic fatigue syndrome.

### REFERENCES

CSIRO Division of Human Nutrition, AND Anti Cancer Foundation of South Australia. The 12345+ Food and Nutrition Plan. Adelaide: Anti Cancer Foundation of South Australia, 1991.
Davidson, G. P.: Lancet, September 23, 1989
Donovan, S.M.J., Zijlstra, R.T., and Odle, J (1994). Use of the Piglet to steady the role of growth factors in National Intestinal Development, Endocrine Regulations, 28, 153-162.
Foley, J. A., D. E. Otterby. Availability, storage, treatment, composition and feeding value of surplus colostrum. J Dairy Sci. 61: 1033-1060, 1978.
Ford, R.P.K., I.S. Menzies, A.D. Phillips, J.A. Walker-Smith and M.W. Turner. Intestinal sugar permeability: relationship to diarrhoeal disease and small bowel morphology. J. Pediatr. Gastroenterol. Nutr. 4: 568-574,1986.
Fryburg, D. A., L. A. Jahn, S. A. Hill, D. M. Oliveras, E. J. Barrett. Insulin and insulin-like growth factor 1 enhance human skeletal muscle protein anabolism during hyperaminoacidemia by different mechanisms. J. Clin. Invest. 96: 1722-1729, 1995.
Marcotty, C. F., J. Frankenne, G. van Beeumen, G. Maghuin-Rogister, and G. Hennen. Insulin-like growth factor I (IGF-1) from cow colostrum: Purification and characterisation. Growth Regulat. 1: 56-61,1991.
Mero, A., H Miikkulainen, J. Riski, R. Pakkanen, J. Aalto, AND T. Takala. Effects of bovine colostrum supplementation on serum IGF-1, IgG, hormone, amino acid and saliva IgA concentrations during training. J. Appl. Physiol., 1997.
M & H Williams Pty Ltd. SERVE Dietary Analysis Software. Sydney.
Oda, S., H. Satoh, T. Sugawara, M. Matsunaga, T. Kuhara, K. Katoh, Y. Shoji, A. Nihel, M. Ohta, Y. Sasaki. Insulin-like growth factor-1, GH, insulin and glucagon concentrations in bovine colostrum and in plasma of dairy cows and neonatal calves around parturition. Comp. Biochem. Physiol. 94A; 805-808, 1989.
Owens, J.A., K.L. Kind, F. Carbone, J.S. Robinson AND P.C. Owens. Circulating insulin-like growth factors-I and -II and substrates in fetal sheep following restriction in placental growth. J. Endocrinol. 140: 5-13, 1994
Owens, P.C., R.J. Johnson, R.G. Campbell, AND F.J. Ballard. Growth hormone increases insulin-like growth factor-I (IGF-1) and decreases IGF-1 in plasma of growing pigs. J. Endocrinol. 124: 269-275, 1990.
Reiter, B. Review of the progress of dairy science: antimicrobial systems in milk. J. Dairy. Res. 45: 131-147, 1978.
Shams, D. Growth factors in milk. Endocr. Regul. 28: 3-8, 1994.
Tomas, F. M., S. E. Knowles, P. C. Owens, L. C. Read, C. S. Chandler, S. E. Gargosky, F. J. Ballard. Effects of full-length and truncated insulin-like growth factor-1 on nitrogen balance and muscle protein metabolism in nitrogen-restricted rats. J. Endocrinol. 128: 97-105, 1991.
Tomas, F. M., S E. Knowles, P. C. Owens, L. C. Read, C. S. Chandler, S. E. Gargosky, F. J. Ballard. Increased weight gain, nitrogen retention and muscle protein synthesis following treatment of diabetic rats with insulin-like growth factor (IGF)-1 and des(1-3)IGF-1. Biochem. J. 276: 547-554, 1991.
Tungthanathanich, P., R. J. Xu; G. W. Reynolds, H. V. Simpson, D. J. Mellor. The effect of milk diets on small intestinal growth in newborn piglets. Proc. Nutr. Soc. NZ. 17: 51-55, 1992.
Zumkeller, W. Relationship between insulin-like growth factor-I and II and IGF-binding proteins in milk and the gastrointestinal tract: growth and development of the gut. J. Paediatr. Gastroenterol. 15: 357-369, 1992.

## Claims

1. A food composition for use in treating or preventing a disorder of the gut, wherein said food composition includes colostrum or a fraction thereof wherein said fraction includes colostrum-derived growth factors maintained therein following fractionation of the colostrum.

2. A food composition according to claim 1 wherein the disorder of the gut is selected from the group including mucositis, gastrointestinal damage from administration of non-steroidal anti-inflammatory drugs, gastrointestinal damage from irradiation therapy, gastrointestinal damage from chemotherapy, damage from infection in normal and in HIV/AIDS patients caused by pathogens selected from the group including rotavirus, *E. Coli* spp, *Salmonella* spp, *Cryptosporidium* spp, *H pylori,* damage from gut surgery, and damage due to disease such as Crohn's disease, inflammatory bowel syndrome, coeliac disease, or cystic fibrosis.

3. A method of improving gut growth and development, said method including administering an effective amount of a food composition wherein said food composition includes colostrum or a fraction thereof wherein said fraction includes colostrum-derived growth factors maintained therein following fractionation of the colostrum.

4. A method of treating short bowel syndrome, said method including administering an effective amount of a food composition wherein said food composition includes colostrum or a fraction thereof wherein said fraction includes colostrum-derived growth factors maintained therein following fractionation of the colostrum.

5. A method of improving the bioavailability of components in colostrum which lead to changed work capacity and/or body composition, said method including administering an effective amount of a food composition wherein said food composition includes colostrum or a fraction thereof wherein said fraction includes colostrum-derived growth factors maintained therein following fractionation of the colostrum.

6. A method of changing body composition and/or physical work capacity, said method including administering an effective amount of a food composition wherein said food composition includes colostrum or a fraction thereof wherein said fraction includes colostrum-derived growth factors maintained therein following fractionation of the colostrum.

7. A method of increasing tissue mass, said method including administering an effective amount of a food composition wherein said food composition includes colostrum or a fraction thereof wherein said fraction includes colostrum-derived growth factors maintained therein following fractionation of the colostrum.

8. A method of increasing fat utilisation, said method including administering an effective amount of a food composition wherein said food composition includes colostrum or a fraction thereof wherein said fraction includes colostrum-derived growth factors maintained therein following fractionation of the colostrum.

9. A method of reducing physiological fatigue and/or physiological perception of that fatigue, said method including administering an effective amount of a food composition wherein said food composition includes colostrum or a fraction thereof wherein said fraction includes colostrum-derived growth factors maintained therein following fractionation of the colostrum.

10. A method of increasing height, said method including administering an effective amount of a food composition wherein said food composition includes colostrum or a fraction thereof wherein said fraction includes colostrum-derived growth factors maintained therein following fractionation of the colostrum.

11. A method of increasing recovery after exercise, said method including administering an effective amount of a food composition wherein said food composition includes colostrum or a fraction thereof wherein said fraction includes colostrum-derived growth factors maintained therein following fractionation of the colostrum.

12. A method of reducing muscle damage during exercise, said method including administering an effective amount of a food composition wherein said food composition includes colostrum or a fraction thereof wherein said fraction includes colostrum-derived growth factors maintained therein following fractionation of the colostrum.

13. A method of increasing physiological buffering capacity, said method including administering an effective amount of a food composition wherein said food composition includes colostrum or a fraction thereof wherein said fraction includes colostrum-derived growth factors maintained therein following fractionation of the colostrum.

14. A method of improving vertical jump performance, said method including administering an effective amount of a food composition wherein said food composition includes colostrum or a fraction thereof wherein said fraction includes colostrum-derived growth factors maintained therein following fractionation of the colostrum.

15. A method of improving the ability to generate peak power and peak force, said method including administering an effective amount of a food composition wherein said food composition includes colostrum or a fraction thereof wherein said fraction includes colostrum-derived growth factors maintained therein following fractionation of the colostrum.

16. A method of increasing endurance exercise performance, said method including administering an effective amount of a food composition wherein said food composition includes colostrum or a fraction thereof wherein said fraction includes colostrum-derived growth factors maintained therein following fractionation of the colostrum.

17. A method of reducing fat mass, said method including administering an effective amount of a food composition wherein said food composition includes colostrum or a fraction thereof wherein said fraction includes colostrum-derived growth factors maintained therein following fractionation of the colostrum.

18. A food composition for use in changing body composition and/or physical work capacity, wherein said food composition includes colostrum or a fraction thereof wherein said fraction includes colostrum-derived growth factors maintained therein following fractionation of the colostrum.

19. A food composition or method according to any one of the preceding claims wherein said food composition further includes casein.

20. A food composition or method according to any one of the preceding claims wherein the growth factor is IGF-1.

21. A food composition or method according to claim 19 or 20 wherein said casein is colostrum-derived and maintained therein following fractionation of the colostrum.

22. A method of producing a food composition including colostrum or a fraction thereof including colostrum-derived growth factors maintained therein following fractionation of the colostrum for use in changing body composition and/or physical work capacity, said method including:
providing colostrum prepared by a process including:
subjecting colostrum to an ultra-filtration process to provide an ultra-filtered colostrum retentate;
subjecting the ultra-filtered colostrum retentate to a spray drying process; and
removing the spray-dried colostrum.

23. A method according to claim 22 further including a bacterial reduction step including centrifuging the colostrum in a flow-through centrifuge wherein the centrifugation is performed by controlling throughput and residence time of the colostrum during centrifugation.

24. A method according to claim 23 further including combining the centrifugation with low heat treatment by subjecting the colostrum to less than 72°C.

25. A food composition prepared by the method according to any one of claims 22 to 24.

26. A food composition or method according to any one of claims 1 to 18 wherein said food composition is prepared by the method according to any one of claims 22 to 24.

27. A food composition or method according to claim 25 or 26 further including casein.

28. A food composition or method according to claim 27 wherein the casein is colostrum derived following fractionation of the colostrum.
